(19) Europäisches Patentamt
European Patent Office
Office européen des brevets

(11) EP 2 724 732 A1

(12) EUROPEAN PATENT APPLICATION
published in accordance with Art. 153(4) EPC

(43) Date of publication:
**30.04.2014 Bulletin 2014/18**

(21) Application number: **12803268.7**

(22) Date of filing: **21.06.2012**

(51) Int Cl.:
***A61L 31/00*** (2006.01) ***A61B 17/00*** (2006.01)

(86) International application number:
**PCT/JP2012/065867**

(87) International publication number:
**WO 2012/176841 (27.12.2012 Gazette 2012/52)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.06.2011 JP 2011140076**

(71) Applicants:
- **Inoue, Kanji**
  **Kyoto-shi, Kyoto 603-8054 (JP)**
- **Toray Industries, Inc.**
  **Tokyo, 103-8666 (JP)**

(72) Inventors:
- **SAKAGUCHI, Hirokazu**
  **Otsu-shi**
  **Shiga 520-8558 (JP)**
- **SAKAGUCHI, Yuka**
  **Otsu-shi**
  **Shiga 520-8558 (JP)**
- **TANAHASHI, Kazuhiro**
  **Otsu-shi**
  **Shiga 520-8558 (JP)**

(74) Representative: **Kador & Partner**
**Corneliusstrasse 15**
**80469 München (DE)**

# (54) INSTRUMENT FOR CAPTURING FREE THROMBI

(57) The present invention aims at providing an instrument for capturing free thrombi that inhibits blood coagulation reaction at the stage of primary hemostasis, in which platelets are involved, and at the stage of coagulation thrombus formation, in which blood coagulation factors are involved, thereby securely capturing free thrombi and extending the available time of the instrument. The present invention provides an instrument for capturing free thrombi including a compound having an antithrombin activity immobilized on a surface(s) thereof; and a method for capturing free thrombi, which method includes capturing free thrombi in blood in a living body using this instrument for capturing free thrombi.

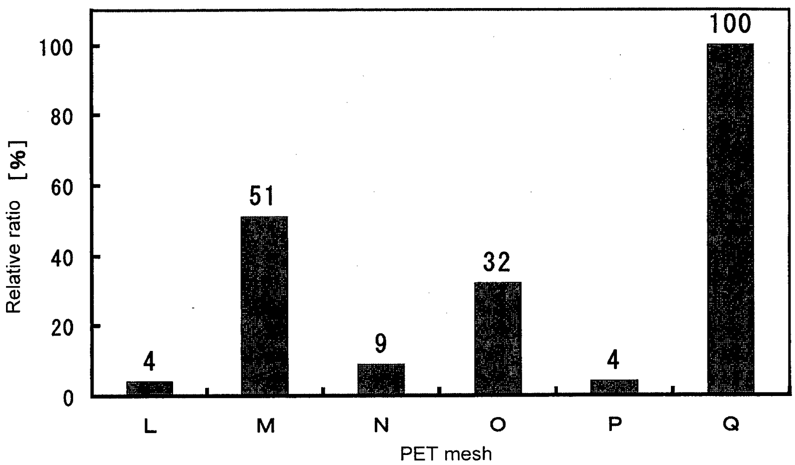


Fig.1



Printed by Jouve, 75001 PARIS (FR)

## Description

### TECHNICAL FIELD

The present invention relates to an instrument for capturing free thrombi.

### BACKGROUND ART

In recent years, treatment of aortic diseases employs percutaneous procedures such as insertion/placement of various instruments including artificial blood vessels and stents in the lesion through a catheter introduced into a human body from a site of incision of an arterial vessel. However, in such percutaneous procedures, there is a risk of releasing of a thrombus from a fragile inner wall of a blood vessel in the lesion to cause clogging of a narrow blood vessel in the distal side, leading to necrosis of a tissue downstream thereof. In particular, clogging of the carotid artery extending to the head with a thrombus may threaten the life.

In order to avoid such a risk, an instrument for capturing free thrombi for capturing thrombi released from a blood vessel, which instrument is transiently placed in the distal side rather than the lesion in which an instrument such as an artificial blood vessel is to be placed, is being developed (Patent Document 1). The instrument for capturing free thrombi comprises a filter section composed of a mesh material or the like in order to capture the released thrombi.

The blood coagulation reaction involved in the formation of a thrombus is a very complex reaction in which various blood coagulation factors are involved, and it has been considered that the stage of primary hemostasis, in which platelets are involved, and the stage of coagulation thrombus formation, in which blood coagulation factors such as thrombin are involved to stabilize and strengthen fibrin, are especially important. No specific compound has been developed that can inhibit the blood coagulation reaction in both the stage of primary hemostasis, in which platelets are involved, and the stage of coagulation thrombus formation, in which blood coagulation factors are involved.

Although the blood coagulation reaction is indispensable for achieving hemostasis upon bleeding caused by injury or the like, there is a danger that contacting of blood with an instrument such as an artificial blood vessel in a percutaneous procedure using the instrument may promote the blood coagulation reaction, causing inhibition of blood flow by formation of a blood clot or coagulation thrombus.

### PRIOR ART DOCUMENTS

[Patent Document]

[Patent Document 1] JP 4073869 B

### SUMMARY OF THE INVENTION

### PROBLEMS TO BE SOLVED BY THE INVENTION

However, at present, since the size of the pores in the filter section of the instrument for capturing free thrombi needs to be as small as possible in order to securely capture free thrombi, blood flow is disturbed to cause congestion, which then promotes the blood coagulation reaction and again causes disturbance of blood flow, resulting in a vicious circle. Because of such a background, even with continuous administration of an anticoagulant to the blood of the patient, the available time of a conventional instrument for capturing free thrombi has been very limited. There is an instrument for capturing free thrombi whose surfaces are coated with an anticoagulant, heparin (Spider FX; ev3), but even this instrument can be used for only not more than 1 hour. Therefore, a percutaneous procedure using an instrument such as an artificial blood vessel needs to be finished within a short period of time, and the burden of the physician who performs the percutaneous procedure is extremely heavy. Thus, a completely novel method is demanded for extending the available time of an instrument for capturing free thrombi.

In view of this, the present invention aims to provide an instrument for capturing free thrombi that inhibits the blood coagulation reaction at the stage of primary hemostasis, in which platelets are involved, and at the stage of coagulation thrombus formation, in which blood coagulation factors are involved, thereby securely capturing free thrombi and extending the available time of the instrument.

MEANS FOR SOLVING THE PROBLEMS

As a result of intensive study to solve the above-described problems, the present inventors discovered that an instrument for capturing free thrombi comprising a compound having an antithrombin activity immobilized on a surface(s) thereof shows a remarkable anticoagulant action, and that the compound having an antithrombin activity is strongly immobilized on the surface(s) of the instrument for capturing free thrombi.

That is, the present invention provides an instrument for capturing free thrombi, comprising a compound having an antithrombin activity immobilized on a surface(s) thereof. Further, the present invention provides the above-described instrument of the present invention for use in capturing free thrombi. Further, the present invention provides a method for capturing free thrombi, the method comprising capturing free thrombi in blood in a living body using the instrument of the present invention.

The compound having an antithrombin activity is preferably immobilized on the surface(s) of the instrument for capturing free thrombi as a conjugate with a macromolecular compound, preferably a macromolecular compound mainly constituted by units derived from at least one type of monomers selected from the group consisting of ethylene glycol, vinyl acetate, vinyl pyrrolidone, propylene glycol, vinyl alcohol and siloxane. That is, the instrument for capturing free thrombi is preferably an instrument for capturing free thrombi comprising a conjugate immobilized on the surface(s), which conjugate is formed between the compound having an antithrombin activity and a macromolecular compound, preferably a compound mainly constituted by units derived from at least one type of monomers selected from the group consisting of ethylene glycol, vinyl acetate, vinyl pyrrolidone, propylene glycol, vinyl alcohol and siloxane.

The compound having an antithrombin activity is preferably a compound represented by the General Formula (I) below:

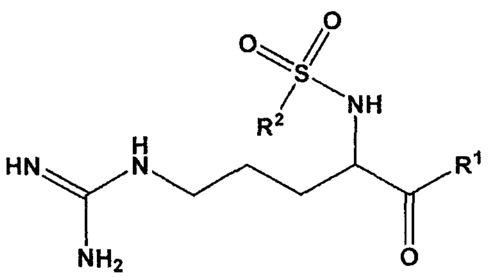

• • • ( I )

[wherein $R^1$ represents a (2R,4R)-4-alkyl-2-carboxypiperidino group, and $R^2$ represents a phenyl group or a fused polycyclic compound residue, which fused polycyclic compound residue is optionally substituted by a lower alkyl group(s) and/or lower alkoxy group(s), and/or by an amino group(s) substituted by a lower alkyl group(s)].

The macromolecular compound is preferably one or more types of compounds selected from the group consisting of polyether-modified silicones, vinyl acetate-vinyl pyrrolidone copolymers and partially saponified polyvinyl alcohols. The macromolecular compound is especially preferably an amino-polyether-modified silicone.

The compound represented by General Formula (I) is preferably (2R,4R)-4-methyl-1-((2*S*)-2-{[(3*RS*)-3-methyl-1,2,3,4-tetrahydroquinolin-8-yl]sulfonyl}amino-5-guanidinopentanoyl)piperidine-2-carboxylic acid.

The instrument for capturing free thrombi preferably comprises a bag-shaped filter section, more preferably comprises: a ring-shaped section; a core section penetrating the ring-shaped section; a bag-shaped filter section whose open end is attached to the ring-shaped section and whose closed end is attached to a part of the distal side of the core section; and a support wire section arranged between the core section and the ring-shaped section.

The material of the filter section is preferably one or more types of compounds selected from the group consisting of polyesters, polyalkyl(meth)acrylates, polyurethanes, polyvinyl chloride and polycarbonate, and the material is especially preferably polyethylene terephthalate.

EFFECTS OF THE INVENTION

The present invention can provide an instrument for capturing free thrombi comprising a compound that remarkably inhibits the blood coagulation reaction at the stage of primary hemostasis, in which platelets are involved, and at the stage of coagulation thrombus formation, in which blood coagulation factors are involved, which compound is strongly immobilized on the surface(s) of the instrument while maintaining its anticoagulant activity. Further, the instrument for capturing free thrombi of the present invention enables secure capturing of free thrombi and remarkable extension of the available time of the instrument, thereby reducing the burden of the physician who performs a percutaneous procedure using an instrument such as an artificial blood vessel.

BRIEF DESCRIPTION OF THE DRAWINGS

Fig. 1 is a diagram showing the relative ratio of platelets attached to each prepared polyethylene terephthalate mesh.
Fig. 2 is a schematic view showing the spread state of an embodiment of the instrument for capturing free thrombi of the present invention.
Fig. 3 is a schematic view showing the folding process of an embodiment of the instrument for capturing free thrombi of the present invention.
Fig. 4 is a schematic view showing the folded state of an embodiment of the instrument for capturing free thrombi of the present invention.

BEST MODE FOR CARRYING OUT THE INVENTION

The terms used in the present description are as defined below unless otherwise specified.
The term "instrument for capturing free thrombi" means a medical instrument also called a filter instrument, and comprises a filter section composed of a mesh material and/or the like for capturing free thrombi.
Examples of the filter section of the instrument for capturing free thrombi include a filter section prepared by forming a sheet having a large number of pores into a bag shape, and a filter section prepared by forming a sheet, interweaved with fibers in the form of a mesh or net, into a bag shape.
Examples of the material of the filter section include polymer materials, for example, polyesters such as polyethylene terephthalate (hereinafter referred to as "PET"); polytetrafluoroethylene; cellulose; cellulose acetate; polycarbonate; polysulfone (hereinafter referred to as "PSf"); polyether sulfone; polyalkyl (meth)acrylates (the alkyl moiety is preferably $C_1$-$C_4$ lower alkyl, especially preferably methyl) such as polymethyl methacrylate (hereinafter referred to as "PMMA"); polyamides; polyvinylidene fluoride; polyvinyl chloride; polyacrylonitrile; polyurethanes; polystyrene; polyethylene; polypropylene; polymethylpentene; and polyimides. Among these, polyesters, polyalkyl(meth)acrylates, polyurethanes, polyvinyl chloride, polycarbonate and polytetrafluoroethylene are preferred, and, because of its high flexibility and in vivo stability, polyesters, especially PET, are more preferred. These materials may be used individually, or two or more of these materials may be used in combination.
In cases where the filter section is composed of an organic fiber prepared with the above-described polymer materials, the filter section has a fiber diameter of preferably not more than 40 $\mu$m, more preferably not more than 35 $\mu$m, still more preferably not more than 30 $\mu$m, in order to obtain a thinner filter section and to allow easy folding of the filter section. The lower limit of the fiber diameter is not limited, and, from the viewpoint of strength, the fiber diameter is usually not less than 20 $\mu$m.
The organic fiber constituting the filter section may be either a monofilament or a multifilament, and is preferably a monofilament since it is smoother and less likely to activate the blood coagulation reaction.
The material of the filter section may also be a metal such as stainless steel or a nickel-titanium alloy in view of the durability and the shape retention property. In cases where a metal is used as the material of the filter section, the conjugate that remarkably inhibits the blood coagulation reaction can be strongly immobilized by, for example, using a coupling agent that can be adsorbed or bound to the metal, or coating the surfaces of the metal with the polymer material.
More specific examples of the constitution of the instrument for capturing free thrombi include:

a constitution in which a flexible wire is bent into a loop shape and both ends of the wire is bundled and fixed on a delivery wire, wherein the open end of a bag-shaped filter section is attached to the ring-shaped wire;
a constitution in which a plurality of flexible wires are shaped into a spindle, and both ends of the wires are fixed at two positions on a delivery wire in the longitudinal direction, wherein the open end of a bag-shaped filter section is attached in the middle of the wires in the longitudinal direction and the closed distal end of the bag-shaped section is attached to the distal ends of the wires; and
a constitution as shown in Figs. 2 to 4, comprising: a ring-shaped section 12 composed of a flexible wire material that can be freely bent and has elastic recoverability, which ring-shaped section 12 has a nearly circular shape; a core section 11 that penetrates the ring-shaped section 12, wherein the shape of the core section 11 is linear and can be flexibly changed; a filter section 13 whose open end is attached in its entirety to the ring-shaped section 12 and whose closed end is attached to a part of the distal end side of the core section 11, which filter section 13 is porous and bag-shaped; and a plurality of support wire sections 14 composed of linear members whose shapes can be flexibly changed, which support wire sections 14 are arranged between a part of the core section 11 proximal to the closed end of the filter section 13 and the ring-shaped section 12; wherein the state of the ring-shaped section 12 changes from a folded state where the ring-shaped section 12 is folded such that a plurality of mountains facing the distal end side of the core section 11 and a plurality of valleys facing the proximal end side of the core section

11 alternately occur and the mountains and valleys are positioned close to each other, to a spread state where the ring-shaped section 12 is spread into a nearly circular shape due to the elastic recoverability of the ring-shaped section itself, and the state of the ring-shaped section 12 also changes from the spread state to the folded state due to tensions of the support wire sections 11 exerted by application of an external force to the support wire sections 14 in the direction that causes bundling of the support wire sections 14 with the core section 11 (Patent Document 1; the instrument 1 for capturing free thrombi, having this constitution, is hereinafter referred to as the "filter instrument A").

With a constitution such as that of the filter instrument A, the spread ring-shaped section 12 is supported by the support wire sections 14, so that the direction of the central axis can be easily adjusted nearly to the direction of blood flow. Therefore, even with a simple structure, free thrombi can be stably and securely captured into the filter section 13 without missing it to the distal side. Further, when the ring-shaped section 12 comes into contact with the inner wall of a blood vessel, the elasticity of the ring-shaped section 12 allows appropriate bending of the ring-shaped section 12 following contraction of the blood vessel, so that free thrombi can be stably and securely captured into the filter section 13 without missing the free thrombi to the distal side. Further, when an external force is directly applied to the ring-shaped section 12 or when the support wire sections 14 are brought close to the core section 11 by an external force while the support wire sections 14 are kept tense, the open end of the ring-shaped section 12 is made narrower and compactly folded, so that the filter instrument A can be appropriately delivered in such a folded state to a predetermined site in a blood vessel by insertion of the instrument into a catheter or the like, while the filter instrument A in the state where thrombi are captured in the filter section 13 can be recovered, without missing the thrombi, by folding the ring-shaped section 12 and thereby narrowing the open end.

Examples of the core section 11 of the filter instrument A include thin, flexible wires made of stainless steel or a nickel-titanium alloy, which is excellent in the elastic recoverability. In such a case, the core section 11 may be constituted by a plurality of wires joined together, but the core section 11 is preferably constituted by a single wire.

Examples of the ring-shaped section 12 of the filter instrument A include a wire that is composed of the same material as the core section 11 and formed into a circular ring shape having a diameter of about 4 to 12 mm.

Examples of the filter section 13 of the filter instrument A include those prepared by forming a flexible, firm, triangular and porous sheet into the form of a conical bag. The size of each pore provided in the filter section 13 is preferably 70 to 200 $\mu$m, and, in view of increasing the accuracy of capturing free thrombi while suppressing the blood coagulation reaction, the sizes of the pores are more preferably uniform. More specifically, the sizes of the pores are preferably within the range of the mean value $\pm$ 20%.

The ratio of the area of the pores provided per unit area of the filter section, the pore ratio, is preferably not less than 30%, more preferably not less than 40%, still more preferably not less than 50%, in view of reducing inhibition of blood flow. The pore ratio is, of course, less than 100%, and, in view of the strength, the pore ratio is usually not more than 90%.

Examples of the support wire section 14 of the filter instrument A include wires made of an appropriate material having high strength, whose tension can be increased by the action of an external force, such as metallic wires, and threads and wires made of polymer materials, that may be the same as, or different from, the material of the core section 11.

The term "compound having an antithrombin activity" means a compound having a high binding affinity to thrombin.

The compound having an antithrombin activity is preferably the "compound represented by General Formula (I)" [wherein $R^1$ represents a (2R,4R)-4-alkyl-2-carboxypiperidino group (the alkyl is preferably lower alkyl), and $R^2$ represents a phenyl group or a fused polycyclic compound residue, which fused polycyclic compound residue is optionally substituted by a lower alkyl group(s) and/or lower alkoxy group(s), and/or by an amino group(s) substituted by a lower alkyl group(s); the lower alkyl is $C_1$-$C_4$ alkyl] described above that is a compound comprising a compound comprising a guanidino structure, more preferably (2*R*,4*R*)-4-methyl-1-((2*S*)-2-{[(3*RS*)-3-methyl-1,2,3,4-tetrahydroquinolin-8-yl]sulfonyl}amino-5-guanidinopentanoyl)piperidine-2-carboxylic acid (hereinafter referred to as "argatroban"). Argatroban is a pharmaceutical compound synthesized in 1978 having the selective antithrombin activity of an arginine derivative. Since argatroban is commercially available as an antithrombotic drug, a commercially available product may be used. The term "having the selective antithrombin activity" herein means to have a high binding affinity to thrombin. Examples of the index for evaluating the antithrombin activity of a compound include the inhibition constant (hereinafter referred to as "Ki") calculated from the Lineweaver-Burk plot based on the absorbance of the test solution. A lower Ki indicates higher binding affinity to thrombin, that is, higher antithrombin activity. Ki is preferably not more than 10 $\mu$M, more preferably not more than 1 $\mu$M, still more preferably not more than 500 nM. A single compound having an antithrombin activity may be used, or two or more compounds having an antithrombin activity may be used in combination.

The compound having an antithrombin activity is preferably immobilized on the surface(s) of the instrument for capturing free thrombi as a conjugate with a macromolecular compound mainly constituted by units derived from at least one type of monomers selected from the group consisting of ethylene glycol, vinyl acetate, vinyl pyrrolidone, propylene

glycol, vinyl alcohol and siloxane. The "conjugate" formed by binding of the compound having an antithrombin activity with the macromolecular compound is preferably hydrophilic in cases where its aqueous solution is prepared. The term "mainly constituted" means that not less than 90 mol%, preferably not less than 95 mol%, still more preferably not less than 98 mol% of the total constitution units (repeating units) constituting the macromolecular compound are constituted by the above-described units, and that a unit(s) other than the above-described units may be contained at a content of not more than 10 mol%, preferably not more than 5 mol%, more preferably not more than 2 mol%, as long as the unit(s) do/does not adversely affect the effect of the present invention. The macromolecular compound is more preferably a copolymer of monomers selected from the group consisting of ethylene glycol, vinyl acetate, vinyl pyrrolidone, propylene glycol, vinyl alcohol and siloxane, especially preferably a copolymer of at least two types of monomers selected from these monomers. The molecular weight of the macromolecular compound is not limited, and usually about 5,000 to 2,000,000, preferably 10,000 to 1,500,000, in terms of the weight average molecular weight.

The term "hydrophilicity" means that a compound is soluble in water, or, even in cases where a compound is insoluble in water, the compound interacts with water molecules by electrostatic interactions and/or hydrogen bonds.

Preferred examples of the macromolecular compound that is bound to the compound having an antithrombin activity to constitute the above-described conjugate, especially the "copolymer of monomers selected from the group consisting of ethylene glycol, vinyl acetate, vinyl pyrrolidone, propylene glycol, vinyl alcohol and siloxane" (hereinafter referred to as an "anti-platelet adhesion copolymer"), include: polyvinyl alcohol; polyvinyl pyrrolidone; polyethylene glycol; polypropylene glycol; and macromolecular compounds composed of polyether and polysiloxane; and copolymers and graft polymers of monomers of these macromolecular compounds and other monomers. The macromolecular compound is preferably a macromolecular compound composed of polyether and polysiloxane, partially saponified polyvinyl alcohol, or a copolymer of vinyl pyrrolidone and vinyl acetate, having high hydrophilicity. Since these are commercially available, a commercially available product may be used. The commercially available products used in the Examples below are examples of the macromolecular compound that can be used in the present invention. These may be used individually, or two or more of these may be used in combination.

Examples of the "macromolecular compound composed of polyether and polysiloxane" include copolymers, polymer complexes and polymer blends of polyether and polysiloxane. The copolymer of polyether and polysiloxane is composed of a polyether unit(s) and a polysiloxane unit(s), and the form of the copolymer may be any of a random copolymer, block copolymer and graft copolymer. A polyether-modified silicone is especially preferred since it has high hydrophilicity.

Example of the "polyether" include structures derived from polyethylene oxide or polypropylene oxide. The "polyether" herein means a structure represented by General Formula (II) (wherein $R^3$ represents an alkyl group having not more than 6 carbon atoms), and the "structure derived from polypropylene glycol" as an example of the polyether means a structure represented by General Formula (III).

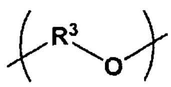

・・・(II)

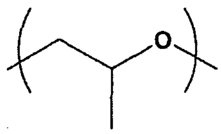

・・・(III)

The "polyether-modified silicone" means a silicone comprising a polyether unit bound to a side chain of the silicone chain, and may be a polyether-modified silicone that is additionally amino-modified or carboxy-modified. The amino group or carboxyl group given by the amino modification or carboxy modification may also be used for covalent bonding with the compound having an antithrombin activity. For example, the amino-modified polyether-modified silicone used in the Examples below (X-22-3939A; Shin-Etsu Chemical) is a preferred example of commercially available hydrophilic polyether-modified silicones.

In cases where the anti-platelet adhesion copolymer is a partially saponified polyvinyl alcohol, the degree of saponification is preferably 50 to less than 100 mol%, more preferably 74 to 99.9 mol%, still more preferably 78 to 95 mol%, in view of ease of handling and achievement of preferred hydrophilicity. The "degree of saponification" herein means the value calculated by Equation 1.

[0045]

$$\text{Degree of saponification} = m/(n+m)\times 100 \quad \ldots \text{ Equation 1}$$

m: number of structures represented by General Formula (IV) in polyvinyl alcohol
n: number of structures represented by General Formula (V) in polyvinyl alcohol

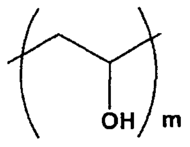

・・・(IV)

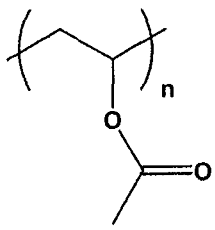

・・・(V)

In cases where the anti-platelet adhesion copolymer is a copolymer of vinyl pyrrolidone and vinyl acetate, the content of vinyl pyrrolidone units is preferably not less than 50 unit mol%, more preferably not less than 60 unit mol%, in view of ease of handling and achievement of preferred hydrophilicity. On the other hand, the content of vinyl pyrrolidone units is preferably less than 100 unit mol% in view of achievement of a preferred amount of immobilization to the instrument for capturing free thrombi. The ratio of vinyl pyrrolidone units in the copolymer of vinyl pyrrolidone and vinyl acetate (unit mol%) can be calculated by subjecting the copolymer to $^1$H-NMR measurement (solvent: $CDCl_3$).

The binding between the compound having an antithrombin activity and the macromolecular compound is preferably achieved by a covalent bond(s) in view of preventing loss of the compound having an antithrombin activity. The covalent bond(s) can be easily formed by performing coupling reaction that forms an amide bond(s) and/or ester bond(s) between a functional group(s) such as a free amino group(s), carboxyl group(s) and/or hydroxyl group(s) in the compound having an antithrombin activity and such a functional group(s) in the macromolecular compound. The coupling reaction can be carried out by, for example, a conventional method using a commercially available well-known coupling agent such as dicyclohexylcarbodiimide (DCC), and the reaction is also specifically described in the Examples below. In cases where the compound having an antithrombin activity is bound via a covalent bond(s), the antithrombin activity needs to be exerted even after the covalent bonding. Whether or not the antithrombin activity is exerted even after the covalent bonding can be investigated by measuring the antithrombin activity of the conjugate after the covalent bonding by the above-described method. In cases where the compound having an antithrombin activity is a compound represented by the above-described General Formula (I), the compound comprises a free amino group at the left end of General Formula (I), and a free carboxyl group in $R^1$. As described in the Examples below, it has been confirmed that the antithrombin activity is still exerted even after binding with the macromolecular compound via the amino group and/or the carboxyl group. In cases where the macromolecular compound is a polyether-modified silicone, a polyether-modified silicone that is additionally amino-modified or carboxy-modified can be used to form an amide bond or ester bond between the amino group or carboxyl group and the free carboxyl group or amino group of the compound of General Formula (I) (Examples below). Further, in cases where the macromolecular compound is a vinyl acetate/polyvinyl pyrrolidone copolymer, an amide bond can be formed between the carboxyl group in the vinyl acetate unit and the amino group in the compound of General Formula (I) (Examples below). Further, in cases where the macromolecular compound is a partially saponified polyvinyl alcohol, an ester bond can be formed between the carboxyl group in the compound of General Formula (I) and the hydroxyl group in the vinyl alcohol unit (Examples below).

The amount of the anti-platelet adhesion copolymer adsorbed on the surface(s) of the filter section of the instrument for capturing free thrombi is preferably not less than 0.1 pg/mm$^2$, more preferably not less than 1 pg/mm$^2$, still more preferably not less than 10 pg/mm$^2$. The upper limit of the amount of adsorption is not restricted, and the amount of adsorption is usually not more than 10 ng/mm$^2$.

The amount of adsorption described above is measured by the following method. First, an untreated sensor chip (Sensor Chip Au; GE Healthcare) is pretreated (distilled water at 25°C; flow rate, 20 μl/min.; 10 minutes) using a surface plasmon resonance apparatus (hereinafter referred to as "SPR") (BIACORE 3000; GE Healthcare), and the

signal value (RU: resonance unit) is measured.

The material for the filter section of the instrument for capturing free thrombi, that is, the target material of immobilization, is dissolved in a solvent, to prepare a 0.5wt% solution of the target material of immobilization. A drop of the solution of the target material of immobilization is added to the center of the gold film area of the pretreated sensor chip attached to a spin coater, and the sensor chip is then immediately coated with the target material of immobilization at room temperature by rotation at 3000 rpm for 1 minute.

After confirming that no droplet is present on the sensor chip, the sensor chip is washed with distilled water using the SPR (25°C; flow rate, 20 μl/min.; 10 minutes), and the sensor chip is then washed 3 times with 0.025 wt% Triton-X100 solution (25°C; flow rate, 20 μl/min.; 1 minute), followed by measuring the signal value 10 minutes after completion of the washing.

Among the thus obtained sensor chips, a sensor chip wherein the difference between the signal values observed before and after the spin coating is within the range of 3000 to 8000 is selected, and the selected sensor chip is washed with distilled water (25°C; flow rate, 20 μl/min.; 10 minutes), followed by 3 times of washing with 0.025 wt% Triton-X100 solution (25°C; flow rate, 20 μl/min.; 1 minute)

Ten minutes after completion of the washing, a solution of the macromolecular compound to be immobilized on the instrument for capturing free thrombi (concentration, 100 μg/ml) is injected (25°C; flow rate, 20 μl/min.; 1 minute), followed by washing with distilled water (25°C; flow rate, 20 μl/min.; 3 minutes). The difference between the signal value observed before beginning of the injection (hereinafter referred to as the "signal value A") and the signal value observed 3 minutes after completion of the injection (hereinafter referred to as the "signal value B") is calculated, and the resulting value is converted according to the following equation: 1 RU=1 $pg/mm^2$.

Subsequently, the sensor chip is washed with distilled water (25°C; flow rate, 20 μl/min.; 2 minutes) and then washed 3 times with 0.025 wt% Triton-X100 solution (25°C; flow rate, 20 μl/min.; 1 minute), further followed by injection of the aqueous solution of the macromolecular compound to be immobilized (concentration, 100 μg/ml) (25°C; flow rate, 20 μl/min.; 1 minute). Thereafter, the same operation is repeated to calculate the signal difference a total of 5 times (difference between the signal value A and the signal value B), and the average of the obtained values is regarded as the amount of adsorption of the anti-platelet adhesion copolymer to the instrument for capturing free thrombi.

Examples of the method for immobilizing the above-described conjugate on the surface(s) of the instrument for capturing free thrombi include a method wherein a solution comprising the conjugate as an effective component(surface treatment agent) is brought into contact with the instrument for capturing free thrombi and then a radiation is irradiated thereto, and a method wherein the conjugate dissolved in an organic solvent is applied or sprayed onto the instrument for capturing free thrombi, followed by drying the instrument. The type of the radiation to be irradiated is preferably an electron beam or γ-ray. The concentration of the macromolecular compound solution to be brought into contact with the surface of the instrument is determined using as an index the antithrombin activity. Examples of the index of the antithrombin activity include the concentration in term of argatroban, and the concentration in term of argatroban of the solution of the conjugate to be brought into contact with the surface of the instrument for capturing free thrombi is about 10 to 200,000 ppm by weight, more preferably about 50 to 100,000 ppm by weight, still more preferably about 1,000 to 100,000 ppm by weight. The conjugate is preferably immobilized on at least the filter section of the instrument for capturing free thrombi.

The instrument for capturing free thrombi of the present invention is placed in a blood vessel of a living body when it is used. The instrument is preferably retained in a catheter, and placed in a blood vessel by insertion of the catheter into the blood vessel.

## EXAMPLES

The present invention is described below in detail by way of Examples, but the present invention is not limited to these.

(Example 1: Binding of Amino-polyether-modified Silicone with Argatroban)

In an eggplant type flask, 5 mmol of argatroban was placed, and 10 mL of anhydrous dimethylformamide (hereinafter referred to as "anhydrous DMF") was added thereto to dissolve the argatroban, followed by adding 10 mL of 4 N hydrochloric acid/1,4-dioxane (Toyo Kasei Co., Ltd.) dropwise to the resulting solution while cooling the eggplant type flask on ice, and then stirring the resulting mixture for 1 hour. Subsequently, the solvent was evaporated with a rotary evaporator, and the resultant was dried overnight in a vacuum drier, followed by adding 25 mL of anhydrous DMF thereto to provide an argatroban hydrochloride/anhydrous DMF solution.

The argatroban hydrochloride/anhydrous DMF solution was placed in a two-necked flask in an amount shown in Table 1, and dicyclohexylcarbodiimide (hereinafter referred to as "DCC")/anhydrous DMF solution and 4-hydroxybenzotriazole (hereinafter referred to as "HOBt")/anhydrous DMF solution were added thereto with stirring under ice-cooling,

followed by further adding an amino-modified polyether-modified silicone (X-22-3939A; Shin-Etsu Chemical) to the resulting mixture and then allowing the reaction to proceed at room temperature for 3 days. Thereafter, the reaction liquid was placed in a dialysis tube (Spectra/Por RC Por 6 MWCO=1000), followed by performing dialysis for 3 days against more than 10 volumes of distilled water while appropriately exchanging the distilled water. The reaction liquid after dialysis was filtered, and the solvent of the obtained filtrate was evaporated with an rotary evaporator, followed by drying the resultant overnight in a vacuum drier, to obtain a conjugate (hereinafter referred to as the "Example 1 conjugate")

(Measurement of Antithrombin activity of Example 1 Conjugate)

The measurement was carried out using ECA-T Kit (HaemoSys). To 100 μL of the Example 1 conjugate, 900 μL of distilled water was added, to prepare an aqueous Example 1 conjugate solution. Thereafter, 30 μL of the aqueous Example 1 conjugate solution was collected and mixed with 100μL of ECA prothrombin buffer and 25 μL of ECA-T substrate, and the resulting mixture was incubated at 37°C for 60 seconds. The mixture was then placed in an apparatus (COATRON M1 (code 80 800 000); Production) and 50 μL of ECA ecarin reagent was further added thereto, followed by performing measurement.

Measurement using the ECA-T kit was carried out in the same manner as described above except that a mixture prepared by mixing 20 μL of an argatroban solution whose concentration was arbitrarily adjusted using an ethanol/hydrochloric acid (volume ratio, 1/4) mixed solvent with 80 μL of human blood plasma, or a mixture prepared by mixing 20 μL of distilled water as a blank with 80 μL of human blood plasma, was used instead of the aqueous Example 1 conjugate solution. A calibration curve was prepared based on the obtained results. The concentration in term of argatroban of the aqueous Example 1 conjugate solution calculated based on the calibration curve, 1494.3 ppm by weight, was regarded as the value indicating the antithrombin activity of the aqueous Example 1 conjugate solution.

(Examples 2 to 13)

The compounds of Example 2 to 13 were obtained in the same manner as Example 1 except that the molar ratios of DCC, HOBt and/or the polyether-modified silicone (X-22-3939A) to the argatroban hydrochloride, and/or the volume ratio of anhydrous DMF to the polyether-modified silicone, were changed. The antithrombic activity was measured for each of these compounds. The molar ratios of DCC, HOBt and the polyether-modified silicone (X-22-3939A) to argatroban hydrochloride, and the result of measurement of the antithrombin activity of each of the compounds of Examples 2 to 13 are shown in Table 1.

[Table 1]

| Compound | Molar ratio to argatroban hydrochloride (1.00) | | | Volume ratio of anhydrous DMF to polyether-modified silicone (1) | Concentration in term of argatroban (ppm by weight) |
|---|---|---|---|---|---|
| | DCC | HOBt | X-22-3939A | | |
| Example 1 | 1.07 | 1.06 | 0.060 | - | 1494.3 |
| Example 2 | 1.04 | 1.04 | 0.060 | - | 831.2 |
| Example 3 | 0.20 | 0.20 | 0.060 | 1.4 | 6610.7 |
| Example 4 | 0.20 | 0.20 | 0.030 | 3.9 | 8393.3 |
| Example 5 | 1.29 | 1.27 | 0.493 | 1.8 | 505.3 |
| Example 6 | 1.29 | 1.27 | 0.203 | 4.3 | 771.7 |
| Example 7 | 1.29 | 1.27 | 0.101 | 8.6 | 606.7 |
| Example 8 | 1.29 | 1.27 | 0.067 | 13.0 | 441.7 |
| Example 9 | 1.29 | 1.27 | 0.049 | 17.6 | 436.7 |
| Example 10 | 1.29 | 1.27 | 0.020 | 42.9 | 738.9 |
| Example 11 | 1.29 | 1.27 | 0.010 | 88.2 | 895.0 |
| Example 12 | 1.00 | 1.00 | 0.060 | - | 6000.0 |
| Example 13 | 1.00 | 1.00 | 0.060 | 40.0 | 5999.4 |

Although the polyether-modified silicone (X-22-3939A) was similarly subjected to measurement of the antithrombin activity, the obtained value was not different from the value for distilled water as the blank, so that it was confirmed that the polyether-modified silicone itself does not have antithrombin activity.

(Measurement of Thrombin Inhibition Constant of Example 1 Conjugate)

In 1 mL of physiological saline, 10,000 U of a bovine thrombin solution (ILS Inc.) was dissolved, to prepare an aqueous bovine thrombin solution.

In 40 mL of distilled water, 25 mg of S-2238 stock solution (Sekisui Medical Co., Ltd.) was dissolved, to prepare an aqueous S-2238 stock solution.

Using a dilution buffer (0.05 M Tris, 0.1 M NaCl, 1 mg/mL bovine serum albumin (BSA), pH 7.4), each of the aqueous bovine thrombin solution, the aqueous S-2238 stock solution, and the aqueous Example 1 conjugate solution described above was diluted.

Into a 96-well plate, 100 μL of the diluted aqueous S-2238 stock solution and 50 μL of the diluted aqueous Example 1 conjugate solution were aliquoted, and the plate was sealed, followed by heating the plate in a constant temperature dryer at 37°C for 30 minutes. Subsequently, 50 μL of the diluted aqueous bovine thrombin solution heated at 37°C for 30 minutes was further aliquoted, and the absorbance was immediately measured using a microplate reader (measurement wavelength, 405 nm, reference wavelength, 595 nm).

After completion of the first measurement of absorbance, the second measurement was immediately carried out. The third and later measurements of absorbance were carried out 4, 6, 8, 10, 12, 14, 16, 18 and 20 minutes after the aliquoting of the bovine thrombin dilution, respectively. From the obtained values of absorbance, Ki was calculated from the Lineweaver-Burk plot. Ki of the Example 1 conjugate was 11.2 nM.

Ki was also calculated for the polyether-modified silicone (X-22-3939A), but Ki of the polyether-modified silicone, which has no antithrombin activity, was the same as that of the blank, as expected.

Further, as a result of similar calculation of Ki for argatroban, Ki was found to be 39.1 nM, which was not less than 3 times higher than Ki of the Example 1 conjugate.

From these results, it is clear that the above-described conjugate has extremely high binding affinity to thrombin, and hence that the conjugate can give remarkable antithrombin activity to the instrument for capturing free thrombi, which activity is even higher than that of argatroban, which is known to have antithrombin activity.

(Example 14: Binding of Vinyl Acetate-Vinyl Pyrrolidone Copolymer to Argatroban)

In a screw bottle, 14.9 g of tetrahydrofuran, 11.5 g of vinyl acetate, 10.8 g of N-vinyl pyrrolidone, 0.028 g of 2-aminoethanethiol and 0.016 g of azobisisobutyronitrile were placed, and the bottle was sealed, followed by irradiation of ultrasonic waves thereto for 10 minutes. The screw bottle was once opened and bubbling with argon gas was performed for 10 minutes, followed by sealing the bottle again. Thereafter, the screw bottle was immersed, with stirring, in a hot water bath at 60°C for 1 hour and then in a hot water bath at 70°C for 6 hours, to allow copolymerization reaction of vinyl acetate and vinyl pyrrolidone. To this reaction liquid, 80 mL of methanol was added, and the resulting mixture was added to about 5 volumes of ether, followed by removal of the supernatant. The operation of adding fresh ether and removing the supernatant was repeated 3 times, and the resultant was dried under reduced pressure, to obtain a vinyl acetate-vinyl pyrrolidone copolymer. The obtained vinyl acetate-vinyl pyrrolidone copolymer was subjected to $^1$H-NMR measurement (solvent; $CDCl_3$), and, as a result, the content of vinyl pyrrolidone units was found to be 60.6 unit mol%.

In 20 mL of anhydrous DMF, 3.58 g of the obtained vinyl acetate-vinyl pyrrolidone copolymer was dissolved, to prepare a vinyl acetate-vinyl pyrrolidone copolymer/anhydrous DMF solution. In a two-necked flask, the whole vinyl acetate-vinyl pyrrolidone copolymer/anhydrous DMF solution prepared and 0.5 mL of argatroban hydrochloride/anhydrous DMF solution (0.49 M) were placed, and 0.5 mL of DCC/anhydrous DMF solution (1.04 M) and 0.5 mL of HOBt/anhydrous DMF solution (1.02 M) were added thereto with stirring under ice-cooling, followed by allowing the reaction to proceed under nitrogen atmosphere at room temperature for 3 days. Subsequently, the reaction liquid was placed in a dialysis tube (Spectra/Por RC Por 6 MWCO=1000), followed by performing dialysis for 3 days against more than 10 volumes of distilled water while appropriately exchanging the distilled water. The reaction liquid after dialysis was filtered, and the solvent of the obtained filtrate was evaporated with a rotary evaporator, followed by drying the resultant overnight in a vacuum drier, to obtain a conjugate (hereinafter referred to as the "Example 14 conj ugate").

(Measurement of Antithrombin activity of Example 14 Conjugate)

In the same manner as the measurement of antithrombin activity of the Example 1 conjugate, measurement was carried out for the Example 14 conjugate/methanol solution (concentration, 20 wt%). The calculated concentration in term of argatroban of the Example 14 conjugate/methanol solution, 104.1 ppm, was regarded as the value indicating

the antithrombin activity of the Example 14 conjugate/methanol solution.

(Immobilization of Example 1 Conjugate to PET Mesh)

Bis-Tris (Dojindo Laboratories) and sodium chloride were dissolved in ultrapure water such that their final concentrations were 0.25 M and 0.5 M, respectively, and 6 N hydrochloric acid was added dropwise to the resulting solution to adjust the pH to 5, to prepare 5 × Bis-Tris buffer.

A PET mesh (fiber diameter, 27 μm; mesh size, 100 μm) was formed into a 6-cm square. The Example 1 conjugate at a concentration in term of argatroban of 50,000 ppm by weight, propylene glycol, 5 × Bis-Tris buffer and distilled water were mixed together at a volume ratio of 8/50/20/22, to obtain a treatment liquid.

The formed PET mesh was rolled into a cylindrical shape and inserted into a polypropylene centrifuge tube, followed by adding 5 mL of the treatment liquid thereto. After irradiation of ultrasonic waves to the tube in a warm bath at 40°C for 1 hour, γ-ray was further irradiated thereto with an absorbed dose of 25 kGy for 3 hours.

Thereafter, the treatment liquid in the centrifuge tube was removed, and 15 mL of 0.025 wt% aqueous polyoxyethylene octylphenyl ether solution was added to the centrifuge tube, followed by shaking the centrifuge tube for 10 minutes to wash the PET mesh. Thereafter, the aqueous solution in the centrifuge tube was removed, and 15 mL of fresh 0.025 wt% aqueous polyoxyethylene octylphenyl ether solution was added to the centrifuge tube, followed by 10 minutes of shaking. This washing operation was repeated a total of 3 times. Subsequently, the same washing operation was repeated 10 times using 15 mL each of distilled water and physiological saline, to prepare a PET mesh to which the Example 1 conjugate was immobilized (hereinafter referred to as the "PET mesh L").

The PET meshes M to P were prepared by the same operation as in the preparation of the PET mesh L except that the treatment liquids prepared with the volume ratios shown in Table 2 were used instead of the above treatment liquid.

[Table 2]

| PET mesh | Mixing volume ratio | | | |
|---|---|---|---|---|
| | Example 1 Compound at concentration in term of argatroban of 50,000 ppm by weight | Propylene glycol | 5 × Bis-Tris buffer | Distilled water |
| L | 8 | 50 | 20 | 22 |
| M | 0.2 | 50 | 20 | 29.8 |
| N | 2 | 30 | 20 | 48 |
| O | 4 | 30 | 20 | 46 |
| P | 16 | 50 | 20 | 14 |

The PET mesh Q was prepared by the same operation as in the preparation of the PET mesh L except that distilled water was used instead of the above treatment liquid.

(Measurement of Amount of Example 1 Conjugate Eluted)

The PET mesh L formed into a 6-mm square was placed in a polystyrene round tube (Code: 352054; BECTON DICKINSON), and 5 mL of human blood plasma was added thereto, followed by shaking the tube for 4 hours. The concentration of the Example 1 conjugate in the human blood plasma after shaking was below the detection limit of the ECA-T kit used for the measurement, and hence no elution of the Example 1 conjugate from the PET mesh L was found. This result indicates that the above conjugate can be strongly immobilized on the instrument for capturing free thrombi.

(Evaluation of Amount of Anti-platelet Adhesion Copolymer Immobilized)

As examples of the copolymer of vinyl pyrrolidone and vinyl acetate (hereinafter referred to as "VA copolymer") to be used as the anti-platelet adhesion copolymer constituting the above conjugate, PVP(K-90), VA73, VA64, VA55 and VA37 (all of these were obtained from BASF) were provided. Similarly, as examples of the partially saponified polyvinyl alcohol to be used as the anti-platelet adhesion copolymer, PVA217, PVA417 and PVA205c (all of these were obtained from Kuraray Co., Ltd.) were provided. Further, as polyether-modified silicones, F114, F244, F303, F3031, F348, F350s, F502, F506 and X-22-3939A (all of these were obtained from Shin-Etsu Silicone, Co., Ltd.) were provided. Each of the VA copolymers, partially saponified polyvinyl alcohols and polyether-modified silicones provided was diluted

with distilled water to prepare its aqueous solution at 10,000 ppm by weight.

On the other hand, for comparison, PEG2000, PEG4000, PEG6000 and PEG20000 (all of these were obtained from Nacalai Tesque); and PEG methyl ether (PEG-em) and PEG dimethyl ether (PEG-dm) (both were obtained from Sigma-Aldrich); were provided as macromolecular compounds that are not included in the anti-platelet adhesion copolymer constituting the above conjugate. Each macromolecular compound provided was diluted with distilled water to prepare its aqueous solution at 10000 ppm by weight.

Binding of argatroban to the above VA copolymer or polyether-modified silicone, binding of argatroban to the partially saponified polyvinyl alcohol, and binding of argatroban to PEG2000, PEG4000, PEG6000 or PEG20000 (all of these were obtained from Nacalai Tesque), or to PEG methyl ether (PEG-em) or PEG dimethyl ether (PEG-dm), were carried out in the same manner as in Example 14 or Example 1.

As examples of the 0.5 wt% solution of the target material of immobilization, on which the anti-platelet adhesion copolymer is to be immobilized, a PMMA (weight average molecular weight, 93000; Sigma-Aldrich)/toluene solution, polyurethane/dimethylacetamide solution, PSf (UDEL (registered trademark), manufactured by Solvay; P-3500)/dimethylacetamide solution, polyvinyl chloride (weight average molecular weight, 80000; Sigma-Aldrich)/tetrahydrofuran solution, polystyrene (Wako)/chloroform solution, and polycarbonate (weight average molecular weight, 20000; TEIJIN Ltd.)/chloroform solution were prepared.

The amount of adsorption of each of the various anti-platelet adhesion copolymers to each target material of immobilization was measured. The results are shown in Table 3.

[Table 3]

| Signal value B - Signal value A $[pg/mm^2]$ | | | | | | | |
|---|---|---|---|---|---|---|---|
| | | Target material of adsorption | | | | | |
| | | PMMA | Polysulfone | Polyurethane | Polyvinyl chloride | Polystyrene | Polycarbonate |
| Macromolecular compound that inhibits adhesion of platelets | PVPK90 | 789 | – | – | – | – | – |
| | VA37 | 2760 | – | – | – | – | – |
| | VA55 | 472 | – | – | – | – | – |
| | VA64 | 920 | – | – | – | – | – |
| | VA73 | 426 | – | – | – | – | – |
| | PVA217 | 2529 | 2886 | 1635 | 2468 | 2777 | 2356 |
| | PVA417 | 2475 | 2742 | 1911 | 2330 | 2662 | 2346 |
| | PVA205c | 2223 | 2130 | 1411 | 1796 | 1989 | 1819 |
| | F114 | 1003 | 844 | 514 | 739 | 621 | 756 |
| | F244 | 1639 | 1272 | 1144 | 1118 | 1052 | 1243 |
| | F303 | 1268 | 1156 | 1604 | 1037 | – | 1374 |
| | F3031 | 947 | 559 | 614 | 418 | 339 | 536 |
| | F348 | 875 | 784 | 756 | 608 | 283 | 800 |
| | F350s | 751 | 657 | 674 | 544 | 275 | 591 |
| | F502 | 827 | 657 | 696 | 385 | 197 | 482 |
| | F506 | 691 | 308 | 437 | 167 | 43 | 279 |
| | X-22-3939A | 1182 | 910 | 1204 | 695 | 924 | 1424 |
| | PEG2000 | 2 | – | – | – | – | – |
| | PEG4000 | 2 | – | – | – | – | – |
| | PEG6000 | 5 | – | – | – | – | – |
| | PEG20000 | 113 | – | – | – | – | – |
| | PEG-me | 5 | – | – | – | – | – |
| | PEG-dm | 67 | – | – | – | – | – |

From the results shown in Table 3, it is clear that the anti-platelet adhesion copolymer constituting the above-described conjugate is not limited to the polyether-modified silicone (X-22-3939A), and that strong immobilization on the instrument for capturing free thrombi is possible.

(Evaluation of Number of Platelets Adhered)

The PET mesh L formed into a 1-cm square was placed in an arbitrary well of a 24-well plate, and 1 mL of phosphate buffered saline (hereinafter referred to as "PBS(-)") was added to the well, followed by incubation of the plate at 37°C for 30 minutes.

Platelet rich plasma (hereinafter referred to as "PRP") was prepared by mixing 3.2 wt% aqueous sodium citrate solution with human volunteer blood at a volume ratio of 1/9 and then centrifuging the resulting mixture at 20°C at 1000 rpm for 15 minutes. The platelet number in PRP was preliminarily measured using Automated Hematology Analyzer XT-1800i (Sysmex Corporation).

The PET mesh L after incubation was transferred to an empty well, and 1 mL of the prepared PRP was added thereto, followed by additional incubation of the mesh at 37°C for 2 hours. This PET mesh L was held with tweezers and placed in another well containing 1 mL of PBS(-), followed by gently washing the mesh. This washing operation was repeated a total of 3 times. The whole PBS(-) used in the washing was collected in an empty well.

To the collected PBS(-), 1 mL of 1 wt% polyoxyethylene octylphenyl ether/PBS(-) was added, and the resulting mixture was incubated at 37°C for 15 minutes.

To an empty well, 200 μL of the incubated solution was collected, and 200 μL of PBS(-) was added thereto. To the resulting mixture, 400 μL of Solution C (a mixture prepared by mixing Solution A with Solution B of LDH Cytotoxicity Detection Kit (manufactured by Takara Bio Inc.) at a volume ratio of 1/45) was added immediately after its preparation, and the well was covered with aluminum foil, followed by leaving the mixture to stand at room temperature for 30 minutes. The reaction was then terminated by addition of 200 μL of 1N HCl (final concentration, 0.2 N).

The reaction liquid after termination of the reaction was subjected to measurement of absorbance at a wavelength of 490 nm using a spectrophotometer.

The same operation was carried out also for the PET meshes M to Q, and each reaction liquid after the termination of reaction was subjected to measurement of absorbance at a wavelength of 490 nm.

The PRP was diluted with PBS(-) to concentrations of 1/10, 1/20, 1/50, 1/100, 1/500 and 1/1000. Each diluted PRP solution was collected into an empty well ofa 24-well plate in an amount of 200 μL, and 200 μL of 1% polyoxyethylene octylphenyl ether /PBS(-) was added thereto, followed by incubating the resulting mixture at 37°C for 15 minutes. Each solution after the incubation was collected into an empty well in an amount of 200 μL, and 400 μL of Solution C immediately after preparation was added to the well. The well was covered with aluminum foil, and the mixture was left to stand at room temperature for 30 minutes. Thereafter, 200 μL of 1 N HCl (final concentration, 0.2 N) was added thereto. The reaction liquids were subjected to measurement of absorbance at a wavelength of 490 nm using a spectrophotometer, to prepare a calibration curve.

From the prepared calibration curve and the absorbance at a wavelength of 490 nm of each of the reaction liquids obtained by the treatment with the PET meshes L to Q, the platelet number in each reaction liquid was calculated. The platelet number in the reaction liquid obtained by the treatment with the PET mesh Q was defined as 100%, in order to calculate the ratios of the platelet numbers in the other reaction liquids (hereinafter referred to as "relative ratios"). The results are shown in Fig. 1.

From the results in Fig. 1, it is clear that the above conjugate is capable of giving remarkable anti-platelet adhesion capacity to the instrument for capturing free thrombi.

(Measurement of Whole Blood Clotting Time)

Blood collected from a volunteer was mixed with citric acid at a volume ratio of 9/1, to prepare citrated blood.

In a cuvette (NON-ACTIVATED CLOTTING TEST KIT), 18 μL of physiological saline was placed, and 14.8 μL of Calcicol was added thereto, followed by further adding 342 μL of the citrated blood to the resulting mixture. The mixture was then subjected to measurement using a Sonoclot coagulation & Platelet Function Analyzer (IMI Corporation), and the obtained ACT ONSET value was regarded as the whole blood clotting time. The whole blood clotting time of the blood collected from the volunteer was 545 seconds.

The same measurement was carried out using each of 2, 10 and 20 μM argatroban solutions (solvent: methanol/hydrochloric acid (volume ratio, 4/1)) instead of physiological saline. As a result, the whole blood clotting time was 531, 746 and 849 seconds, respectively.

The same measurement was carried out using each of 0.3, 1.3 and 2.5 μM aqueous Example 1 conjugate solutions instead of physiological saline. As a result, the whole blood clotting time was 527, 693 and 730 seconds, respectively.

(Preparation of Instrument for capturing free thrombi)

A nitinol wire was bent and turned a plurality of times into a ring shape, to prepare a ring-shaped section 12

that is a circular ring having a diameter of 6 mm. A polyester mesh sheet having a 100-μm mesh size was formed into a conical-bag shape having a height of 15 mm, to prepare a filter section 13. In this case, the bottom of the cone corresponds to the open end 13a, and the top corresponds to the closed end 13b.

As the core section 11 (including an operation member 15), a stainless-steel wire was used. At one end of the wire, a guide section 11b composed of a flexible wire gently curving to form an arc was formed.

The core section 11 was made to penetrate the ring-shaped section 12, and the closed end 13b of the filter section 13 was attached to the core section 11 at a position in the proximal side of the guide section at the distal end of the core section 11. Further, the open end 13a of the filter section 13 was attached to the ring-shaped section 12. Four threads made of a synthetic resin or composite fiber, such as polyarylate threads, were provided, and each of these was used as a support wire section 14. A position on the core section 11 that is proximal to the ring 12 was used as a support section 14a, and each of the positions defined by dividing the circumference of the ring-shaped section 12 into four equal parts was defined as a support section 14b. As shown in Fig. 2, each of the four support wire sections 14 was fixed to the support section 14a and the support section 14b by adhesion or the like, to complete a filter instrument A. In the ring-shaped section 12, as shown in Fig. 2, a dividing point $12_1$, dividing point $12_2$, dividing point $12_3$ and dividing point $12_4$ were set at the midpoints of the support sections 14b, and a folding property was preliminarily given to the ring-shaped section 12 such that the dividing point $12_1$ and the dividing point $12_3$, a pair of the dividing points facing each other, become the bottoms of valleys when these dividing points are bent by an external force in the direction of the proximal end 15a of the core section, while the dividing point $12_2$ and the dividing point $12_4$, the other pair of the dividing points, become the tops of mountains when these dividing points are bent by an external force in the direction of the distal end of the core section, that is, such that the ring-shaped section 12 shows a wavy pattern as a whole after folding.

The state of the filter instrument A changes from the spread state shown in Fig. 2 to the folded state by a process in which the support wire sections 14 are bundled with the core section 11 as shown in Fig. 3 by the action of an external force to cause bundling of the support sections 14b with the core section, which makes the two pairs of dividing points that face each other, that is, the pair of the dividing point $12_1$ and the dividing point $12_3$, and the pair of the dividing point $12_2$ and the dividing point $12_4$, become the bottoms of valleys and the tops of mountains, respectively, while the filter section 13 is folded as the ring-shaped section 12 is gradually folded until the paired dividing points come into contact with each other by the folding of the ring-shaped section 12 as shown in Fig. 4. In the folded state, the open end 13a of the filter section 13 is almost completely closed.

(Immobilization of Example 1 Conjugate on Instrument for capturing free thrombi)

The Example 1 conjugate at a concentration in term of argatroban of 50,000 ppm by weight, propylene glycol, 5 × Bis-Tris buffer and distilled water were mixed together at a volume ratio of 8/50/20/22, to obtain a treatment liquid.

In a container with an appropriate capacity, 2 mL of the treatment liquid was placed, and the filter section of the prepared filter instrument A was completely immersed therein, followed by irradiation of ultrasonic waves to the filter section in a warm bath at 40°C for 1 hour and then irradiation of γ-ray with an absorbed dose of 5 kGy for 3 hours.

Thereafter, the filter instrument A was transferred to a polypropylene centrifuge tube containing 15 mL of 0.025 wt% aqueous polyoxyethylene octylphenyl ether solution, and the 10-cm portion at the was immersed in the aqueous solution, followed by leaving the instrument to stand for 10 minutes. Thereafter, the aqueous solution in the centrifuge tube was removed, and 15 mL of fresh 0.025 wt% aqueous polyoxyethylene octylphenyl ether solution was added thereto, followed by leaving the instrument to stand for 10 minutes. This washing operation was repeated a total of 4 times. Subsequently, the same washing operation was repeated 10 times using 15 mL each of distilled water and physiological saline, and the instrument was then subjected to drying under reduced pressure and EOG sterilization, to prepare a filter instrument A on which the Example 1 conjugate was immobilized (hereinafter referred to as the "instrument for capturing free thrombi X").

As a control experiment, a filter instrument A separately prepared was subjected to only EOG sterilization, to provide a filter instrument A to which the Example 1 conjugate is not immobilized (hereinafter referred to as the "instrument for capturing free thrombi Y").

(In Vivo Placement Test)

The instrument for capturing free thrombi X was contracted to a diameter of 3 Fr, and stored in a catheter. A dog (hybrid beagle) was subjected to measurement of the whole blood clotting time (hereinafter referred to as "ACT"), and 1500 units of a heparin injection was administered to the dog. Thereafter, ACT was measured again to confirm that ACT was within the range of 200 to 300 s.

Into the above dog, a 7-Fr sheath catheter was inserted, and a 6-Fr guide catheter was further inserted, followed by administration of a contrast medium to determine the blood vessel where the instrument for capturing free thrombi

X was to be placed. The instrument for capturing free thrombi X stored in the catheter was then delivered to the carotid artery of the dog (blood vessel diameter, 6 mm). The filter section of the instrument for capturing free thrombi X delivered to the desired site was opened to begin indwelling of the instrument for capturing free thrombi X. Thereafter, a contrast medium was administered to see whether or not blood was passing through the filter section of the instrument for capturing free thrombi X. Administration of an anticoagulant, such as heparin, was not carried out at all.

ACT of the dog became a normal value about 2 hours after the administration of a heparin injection. However, as a result of the test, inhibition of blood flow by the placed instrument for capturing free thrombi X was not observed, and blood was capable of stably passing through the instrument for capturing free thrombi X for not less than 5 hours after the beginning of indwelling.

The same operation as described above was carried out except that the instrument for capturing free thrombi C was used instead of the instrument for capturing free thrombi X, to see whether or not blood was passing through the filter section of the instrument for capturing free thrombi Y. As a result of the test, inhibition of blood flow by the instrument for capturing free thrombi Y was found, and the filter section of the instrument for capturing free thrombi Y was occluded 15 minutes after the beginning of indwelling. On the filter section of the instrument for capturing free thrombi Y recovered, formation of thrombi and membranous deposits was found.

The same operation as described above was carried out except that the instrument for capturing free thrombi Y was used instead of the instrument for capturing free thrombi X, and that heparin was continuously administered during the test, to see whether or not blood was passing through the filter section of the instrument for capturing free thrombi Y. As a result of the test, inhibition of blood flow by the instrument for capturing free thrombi Y was found even with the continuous administration of heparin, and the filter section of the instrument for capturing free thrombi Y was occluded 30 minutes after the beginning of indwelling. On the filter section of the instrument for capturing free thrombi Y recovered, formation of thrombi and membranous deposits was found.

(Example 15)

In an eggplant type flask, 44.8 mmol of argatroban was placed, and 50 mL of anhydrous dimethylformamide (hereinafter referred to as "anhydrous DMF") was added thereto under Ar gas flow to dissolve argatroban, followed by cooling the eggplant type flask on ice. To the resulting solution, 50 mL of 4 N hydrochloric acid/1,4-dioxane (Toyo Kasei Co., Ltd.) was added dropwise, and the resulting mixture was stirred at room temperature for 1 hour. Subsequently, the solvent was evaporated with a rotary evaporator, and the resultant was subjected to azeotropic distillation treatment with anhydrous toluene (Wako). The resultant was further dried overnight in a vacuum drier, and anhydrous DMF was added to the obtained compound, to provide an argatroban hydrochloride/anhydrous DMF solution (1.0 M).

In a three-necked flask, 46 ml of the argatroban hydrochloride/anhydrous DMF solution was placed, and 57.8 mmol of HOBt and 20 ml of anhydrous DMF were added thereto with stirring under ice-cooling. After dissolving the reagent, 51.0 mmol of DCC was added thereto. To 190 g of amino-polyether-modified silicone (X-22-3939A; Shin-Etsu Chemical) preliminarily dried under reduced pressure at 40°C for 5 hours, 760 g of anhydrous DMF was added, and the resulting mixture was stirred. The solution of argatroban hydrochloride, DCC and HOBt in anhydrous DMF was added to the amino-polyether-modified silicone/anhydrous DMF solution under ice-cooling. After repeating degassing and replacement of the atmosphere with Ar 5 times, the mixture was allowed to react at room temperature for 3 days with stirring. The reaction liquid was placed in a dialysis tube (Spectra/Por RC Por 6 MWCO=15,000), followed by performing dialysis for 7 days against more than 100 volumes of distilled water while appropriately exchanging the distilled water. The reaction liquid after dialysis was filtered, and the solvent of the obtained filtrate was evaporated with a rotary evaporator, followed by drying the resultant overnight in a vacuum drier, to obtain a conjugate (hereinafter referred to as the "Example 15 conjugate").

(Measurement of Antithrombin activity of Example 15 Conjugate)

The measurement was carried out using ECA-T Kit (HaemoSys). To 10 mg of the Example 15 conjugate, 1 ml of distilled water was added, to prepare an aqueous Example 15 conjugate solution. Thereafter, 30 μL of the aqueous Example 15 conjugate solution was collected and mixed with 100μL of ECA prothrombin buffer and 25 μL of ECA-T substrate, and the resulting mixture was incubated at 37°C for 60 seconds. The mixture was then placed in an apparatus (COATRON M1 (code 80 800 000); Production) and 50 μL of ECA ecarin reagent was further added thereto, followed by performing the measurement.

Measurement using the ECA-T kit was carried out in the same manner as described above except that a mixture prepared by mixing 20 μL of an argatroban solution whose concentration was arbitrarily adjusted using an ethanol/hydrochloric acid (volume ratio, 1/4) mixed solvent with 80 μL of human blood plasma, or a mixture prepared by mixing 20 μL of distilled water as a blank with 80 μL of human blood plasma, was used instead of the aqueous Example 15 conjugate solution. A calibration curve was prepared based on the obtained results. The concentration in term of argatroban of the aqueous Example 15 conjugate solution calculated based on the calibration curve, 2.6 ppm by weight, was regarded as the value indicating the antithrombin activity of the aqueous Example 15 conjugate solution.

(Immobilization of Example 15 Conjugate to Instrument for capturing free thrombi)

The Example 15 conjugate at a concentration in term of argatroban of 344 ppm by weight, propylene glycol, 5 × Bis-Tris buffer and distilled water were mixed together at a volume ratio of 10/50/20/20, to obtain a treatment liquid.
In a container with an appropriate capacity, 2 mL of the treatment liquid was placed, and the filter section of the prepared filter instrument B was completely immersed therein, followed by irradiation of ultrasonic waves to the filter section in a warm bath at 40°C for 1 hour and then irradiation of γ-ray with an absorbed dose of 5 kGy for 3 hours.
Thereafter, the filter instrument B was transferred to a polypropylene centrifuge tube containing 15 mL of 0.025 wt% aqueous polyoxyethylene octylphenyl ether solution, and the 10-cm portion at the distal end was immersed in the aqueous solution, followed by leaving the instrument to stand for 10 minutes. Thereafter, the aqueous solution in the centrifuge tube was removed, and 15 mL of fresh 0.025 wt% aqueous polyoxyethylene octylphenyl ether solution was added thereto, followed by leaving the instrument to stand for 10 minutes. This washing operation was repeated a total of 4 times. Subsequently, the same washing operation was repeated 10 times using 15 mL each of distilled water and physiological saline. The instrument was then further immersed in 15 ml of distilled water for 30 minutes, and subjected to drying under reduced pressure and EOG sterilization, to prepare a filter instrument B to which the Example 15 conjugate was immobilized (hereinafter referred to as the "instrument for capturing free thrombi Z").

(In Vivo Placement Test)

The instrument for capturing free thrombi X was contracted to a diameter of 3 Fr, and stored in a catheter. A dog (hybrid beagle) was subjected to measurement of the whole blood clotting time (hereinafter referred to as "ACT"), and a heparin injection was administered to the dog. Thereafter, ACT was measured again to confirm that ACT was not less than 300 s.
To the above dog, a 7-Fr sheath catheter was inserted, and a 6-Fr guide catheter was further inserted, followed by administration of a contrast medium to determine the blood vessel where the instrument for capturing free thrombi X was to be placed. The instrument for capturing free thrombi Z stored in the catheter was then delivered to the carotid artery of the dog (blood vessel diameter, 6 mm). The filter section of the instrument for capturing free thrombi Z delivered to the desired site was opened to begin indwelling of the instrument for capturing free thrombi Z. Thereafter, a contrast medium was administered to see whether or not blood was passing through the filter section of the instrument for capturing free thrombi X. During the test, an anticoagulant such as heparin was administered to maintain an ACT of not less than 300 s.
As a result of the test, inhibition of blood flow by the placed instrument for capturing free thrombi Z was not observed, and blood was capable of passing through the instrument for capturing free thrombi Z for not less than 5 hours after the beginning of indwelling.
From these results, it is clear that the above conjugate is capable of giving remarkable anticoagulant action to the instrument for capturing free thrombi.

INDUSTRIAL APPLICABILITY

The present invention can be used as an instrument for capturing free thrombi having excellent anticoagulant action in the field of medicine.

DESCRIPTION OF SYMBOLS

1 Instrument for capturing free thrombi
11 Core section
12 Ring-shaped section
13 Filter section
14 Support wire section

## Claims

1. An instrument for capturing free thrombi, comprising a compound having an antithrombin activity immobilized on a surface(s) thereof.

2. The instrument for capturing free thrombi according to claim 1, wherein a conjugate(s) between said compound having an antithrombin activity and a macromolecular compound is(are) immobilized on the surface(s).

3. The instrument for capturing free thrombi according to claim 2, wherein said macromolecular compound is composed of units derived from at least one type of monomers selected from the group consisting of ethylene glycol, vinyl acetate, vinyl pyrrolidone, propylene glycol, vinyl alcohol and siloxane.

4. The instrument for capturing free thrombi according to claim 2 or 3, wherein said macromolecular compound is one or more types of compounds selected from the group consisting of polyether-modified silicones, vinyl acetate-vinyl pyrrolidone copolymers and partially saponified polyvinyl alcohols.

5. The instrument for capturing free thrombi according to claim 4, wherein said macromolecular compound is an amino-polyether-modified silicone.

6. The instrument for capturing free thrombi according to any one of claims 1 to 5, wherein said compound having an antithrombin activity is a compound represented by the General Formula (I) below:

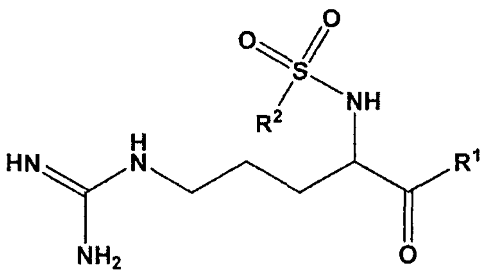

・・・(I)

[wherein $R^1$ represents a (2R,4R)-4-alkyl-2-carboxypiperidino group, and $R^2$ represents a phenyl group or a fused polycyclic compound residue, said fused polycyclic compound residue being optionally substituted by a lower alkyl group(s) and/or lower alkoxy group(s), and/or by an amino group(s) substituted by a lower alkyl group(s)].

7. The instrument for capturing free thrombi according to claim 6, wherein the compound of General Formula (I) is (2*R*,4*R*)-4-methyl-1-((2*S*)-2-{[(3*RS*)-3-methyl-1,2,3,4-tetrahydroquinolin-8-yl]sulfonyl}amino-5-guanidinopentanoyl)piperidine-2-carboxylic acid.

8. The instrument for capturing free thrombi according to any one of claims 1 to 7, comprising a bag-shaped filter section, said filter section being coated with said conjugate(s) between said compound having an antithrombic activity and said macromolecular compound.

9. The instrument for capturing free thrombi according to claim 8, comprising: a ring-shaped section; a core section penetrating said ring-shaped section; a bag-shaped filter section whose open end is attached to said ring-shaped section and whose closed end is attached to a part of the distal end side of said core section; and a support wire section arranged between said core section and said ring-shaped section.

10. The instrument for capturing free thrombi according to claim 8 or 9, wherein the material of said filter section is one or more types of compounds selected from the group consisting of polyesters, polyalkyl(meth)acrylates, polyurethanes, polyvinyl chloride and polycarbonate.

11. The instrument for capturing free thrombi according to claim 10, wherein the material of said filter section is polyethylene terephthalate.

12. The instrument for capturing free thrombi according to any one of claims 8 to 11, wherein said filter section is

irradiated with radiation after being coated with said conjugate(s) between said compound having an antithrombin activity and said macromolecular compound.

**13.** The instrument according to any one of claims 1 to 12, for use in capturing free thrombi.

**14.** A method for capturing free thrombi, said method comprising capturing free thrombi in blood in a living body using the instrument according to any one of claims 1 to 12.

**15.** The method according to claim 14, wherein said instrument is retained in a catheter, which catheter is inserted into a blood vessel.

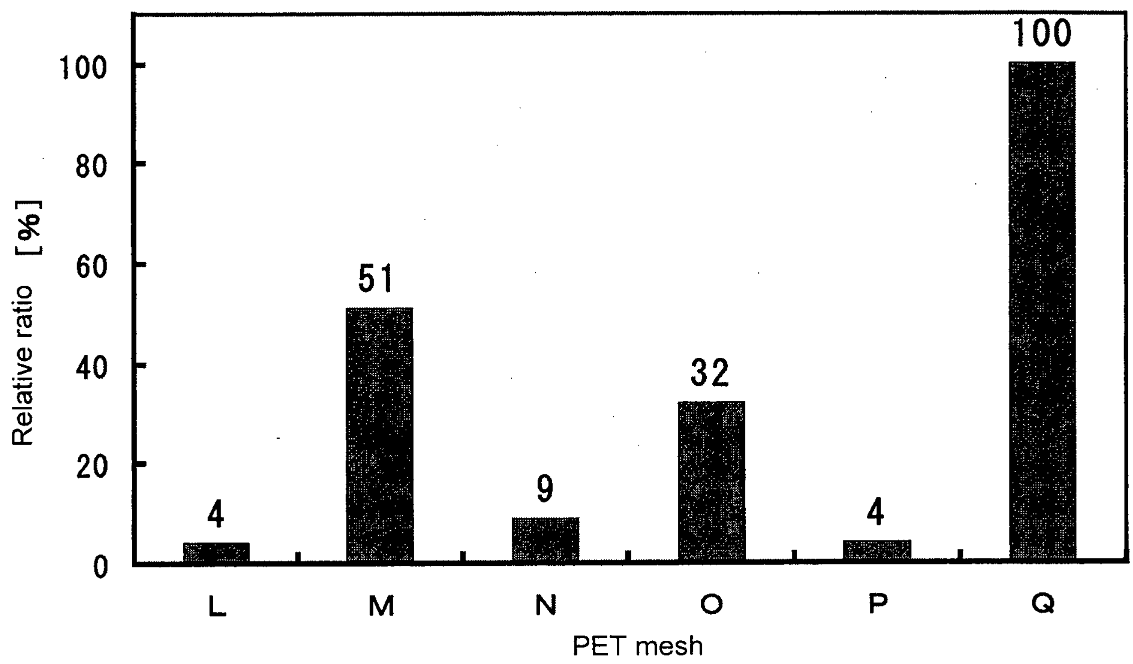
100
80
60
40
20
0
Relative ratio [%]
4
51
9
32
4
100
L
M
N
O
P
Q
PET mesh

Fig.1

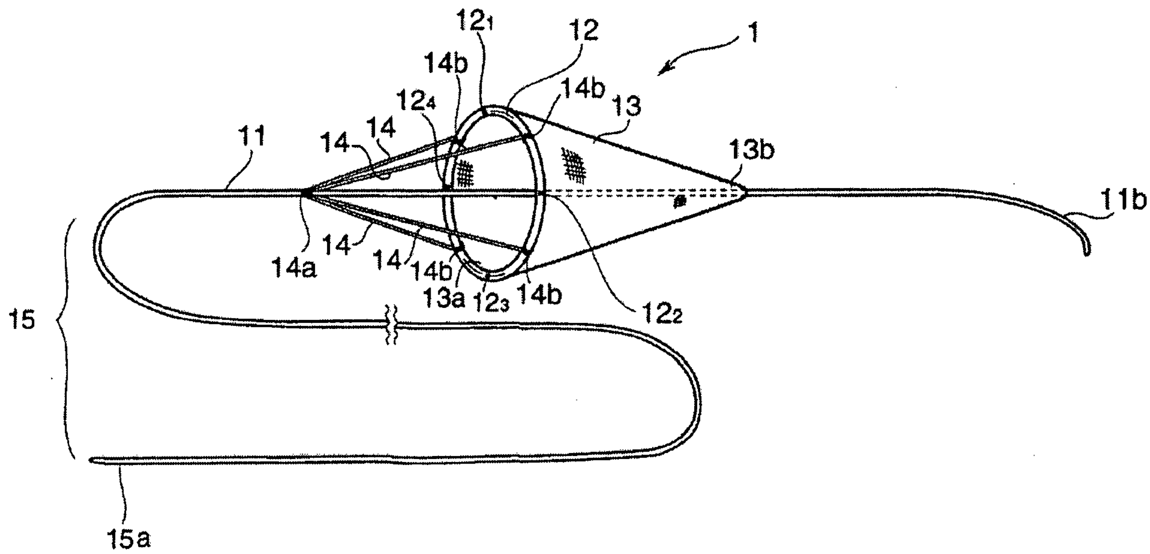
12₁
12
1
14b
12₄
14b
13
14
11
14
13b
11b
14a
14
14
14b
13a
12₃
14b
12₂
15
15a

Fig.2

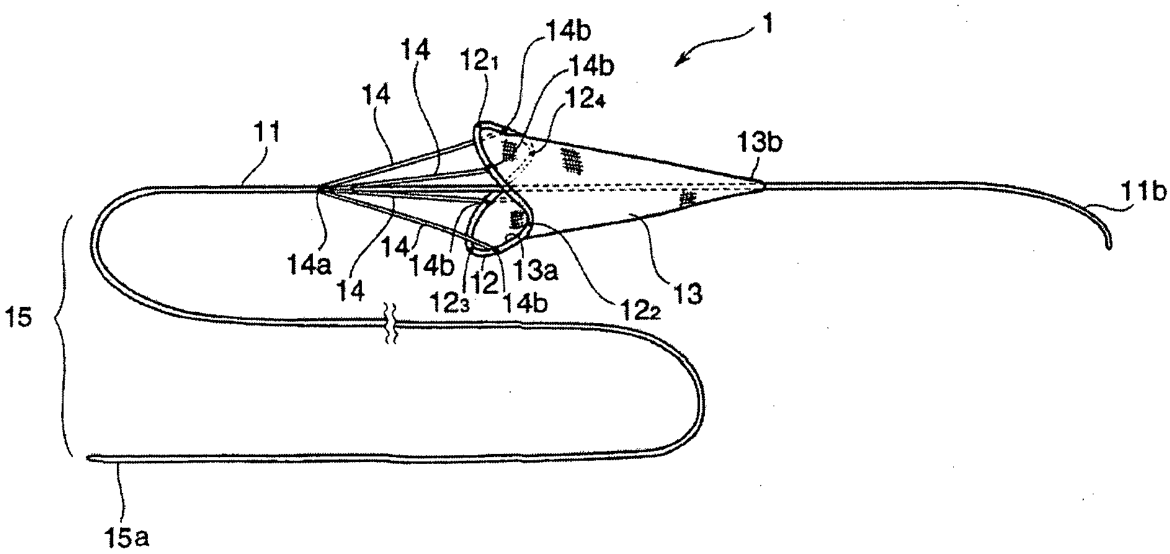
1
14b
14
$12_1$
14b
14
$12_4$
11
13b
11b
14a
14
14b
13a
14
12
15
$12_3$
14b
$12_2$
13
15a

Fig.3

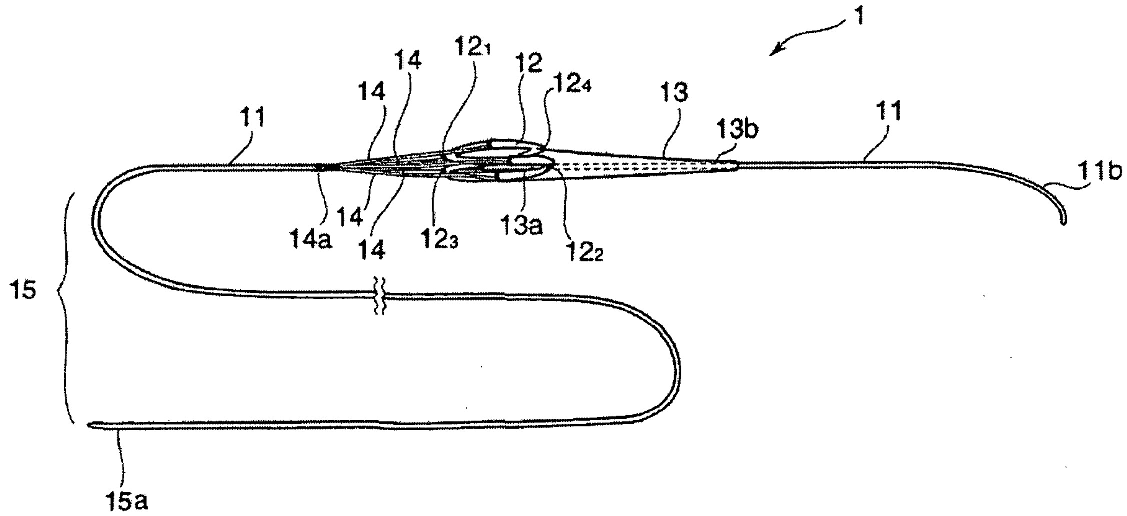
1
14
$12_1$
12
14
$12_4$
13
11
13b
11
11b
14
14a
14
$12_3$
13a
$12_2$
15
15a

Fig.4

**INTERNATIONAL SEARCH REPORT**

International application No.
PCT/JP2012/065867

A. CLASSIFICATION OF SUBJECT MATTER
*A61L31/00*(2006.01)i, *A61B17/00*(2006.01)i

According to International Patent Classification (IPC) or to both national classification and IPC

B. FIELDS SEARCHED

Minimum documentation searched (classification system followed by classification symbols)
A61L31/00, A61B17/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2012 |
| Kokai Jitsuyo Shinan Koho | 1971-2012 | Toroku Jitsuyo Shinan Koho | 1994-2012 |

Electronic data base consulted during the international search (name of data base and, where practicable, search terms used)
CA/REGISTRY/MEDLINE/EMBASE/BIOSIS(STN), JSTPlus/JMEDPlus/JST7580(JDreamII)

C. DOCUMENTS CONSIDERED TO BE RELEVANT

| Category* | Citation of document, with indication, where appropriate, of the relevant passages | Relevant to claim No. |
|---|---|---|
| Y | WO 2003/030740 A1 (Kanji INOUE), 17 April 2003 (17.04.2003) & JP 4073869 B2 & EP 1430839 A1 & US 2004/243173 A1 | 1-13 |
| Y | Masahito SAKUMA et al., "Inferior Vena Cava Filter for Acute Pulmonary Embolism", Jomyakugaku, 2008, vol.19, no.1, pages 23 to 28 | 1-13 |
| Y | JP 2003-024452 A (Kawasumi Laboratories, Inc.), 28 Januarly 2003 (28.01.2003), (Family: none) | 1-13 |
| Y | JP 2009-225824 A (Toray Industries, Inc.), 08 October 2009 (08.10.2009), (Family: none) | 1-13 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 08 August, 2012 (08.08.12) | 21 August, 2012 (21.08.12) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.
PCT/JP2012/065867

C (Continuation). DOCUMENTS CONSIDERED TO BE RELEVANT

| Category* | Citation of document, with indication, where appropriate, of the relevant passages | Relevant to claim No. |
|---|---|---|
| Y | TSUKAGOSHI Tatsuya. et al, Protein adsorption on polymer-modified silica particle surface Colloids Surf B., 2007, Vol.54 No.1 p.101-107 | 1-13 |
| Y | Akitaka SENUMA, Hiroaki SHOJI, "Silicone aiming at new era. Technological development of modified silicone polymers", JETI., 1995, vol.43, no.12, pages 109 to 111 | 1-13 |
| A | Tomoyuki KUNISHIMA et al., "A Case of Anti-phospholipid Antibody Syndrome with Pulmonary Embolism Treated by a Temporary Inferior Vena Cava Filter and Anticoagulant Therapy", Kokyu to Junkan, 2002, vol.50, no.11, pages 1167 to 1171 | 1-13 |
| P,Y | WO 2011/078208 A1 (Toray Industries, Inc.), 30 June 2011 (30.06.2011), (Family: none) | 1-13 |

Form PCT/ISA/210 (continuation of second sheet) (July 2009)

**INTERNATIONAL SEARCH REPORT**

International application No.
PCT/JP2012/065867

**Box No. II Observations where certain claims were found unsearchable (Continuation of item 2 of first sheet)**

This international search report has not been established in respect of certain claims under Article 17(2)(a) for the following reasons:

1. ☒ Claims Nos.: 14,15
because they relate to subject matter not required to be searched by this Authority, namely:
The inventions set forth in the above-said claims pertain to methods for treatment of the human body by therapy or to diagnostic methods.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.
2. ☐ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 4073869 B